# EUROPEAN PATENT APPLICATION

(11) **EP 1 281 379 A2**
(43) Date of publication of application: **05.02.2003**
(21) Application number: 02255216.0
(22) Date of filing: 26.07.2002
(51) Int. Cl.: A61F 13/496, A61F 13/49, A61F 13/491

(54) **Pants-type disposable diaper**

(30) Priority: 30.07.2001 JP 2001229260
(71) Applicant: UNI-CHARM CORPORATION, Kawanoe-shi Ehime-ken (JP)
(72) Inventor: Ishikawa, Hiroki, Technical Center, Mitoyo-gun, Kagawa-ken 769-1602 (JP); Kinoshita, Akiyoshi, Technical Center, Mitoyo-gun, Kagawa-ken 769-1602 (JP)
(74) Representative: Parry, Christopher Stephen

(57) **Abstract**

A pants-type disposable diaper 1 includes a chassis S1 and a liquid-absorbent member 4. The chassis is shaped in a pants-type and has a waist-hole 8 and a pair of leg-holes 9. Elastic members 18 extending in a longitudinal direction of the diaper 1 are arranged between a longitudinal center line bisecting a width of the liquid-absorbent member 4 in the waist-surrounding direction and respective side edges 4b of the liquid-absorbent member 4 so that a zone extending between the elastic members 18 on the liquid-absorbent member 4 is convexed toward the side facing away from a wearer's body as the elastic members 18 contract and this zone 19 defines a penis receiving zone 20 adapted to receive the wearer's penis as the diaper 1 is worn.

## Description

This invention relates to a pants-type disposable diaper for absorption and containment bodily discharges.

A conventional pants-type disposable diaper comprises a liquid-pervious topsheet facing a wearer's body, a liquid-impervious backsheet facing a garment and a liquid-absorbent core interposed between the top- and backsheets. Front and rear waist regions of the top- and backsheets are joined to each other in the vicinity of transversely opposite side edges extending in a longitudinal direction at a plurality of heat-sealing spots arranged intermittently in the longitudinal direction so as to define a waist-hole and a pair of leg-holes below the waist-hole. Such a diaper is disclosed, for example, in Japanese Patent Publication Nos. 1998-71173A and 1998-75980A.

These diapers disclosed in the above-cited Publications are commonly characterized in that a plurality of waist-surrounding elastic members extending in a waist-surrounding direction are attached in a stretched state to respective ends of the front and rear waist regions defining the waist-hole and a plurality of thigh-surrounding elastic members extending in a thigh-surrounding direction are attached in a stretched state to peripheral edge portions of the respective leg-holes. When the diaper is worn, the waist-surrounding elastic members constrict a region around a wearer's torso and the thigh-surrounding elastic members constrict a region around wearer's thighs to prevent the diaper from slipping down and at the same time prevent bodily discharges from leaking through the waist-hole's peripheral edge and the leg-holes' peripheral edges.

However, it is difficult with such a conventional diaper as those disclosed in the above-cited Publications for the wearer to keep a position of his penis in a transversely middle area of the front waist region and the crotch region of the diaper as the wearer' s penis is rather free to move within the diaper. Upon occurrence of urination with the wearer's penis turning toward any one of the leg-holes' peripheral edges, urine may be leaked through this peripheral edge.

It is an object of this invention to provide a pants-type disposable diaper improved so that a wearer's penis can be retained in a transversely middle zone of a front waist region and a crotch region of the diaper.

According to this invention, there is provided a pants-type disposable diaper comprising a chassis defining front and rear waist regions opposed to each other and a crotch region extending between the waist regions, and a liquid-absorbent member extending across the crotch region into the front and rear waist regions, the chassis having longitudinally opposite end portions lying outside longitudinally opposite ends of the liquid-absorbent member extending across the front and rear waist regions in a waist-surrounding direction and transversely opposite side portions lying outside transversely opposite side edges of the liquid-absorbent member extending across the front and rear waist regions in the longitudinal direction, and the front and rear waist regions being joined to each other in a vicinity of side edges contiguous to the opposite side portions forming a waist-hole and a pair of leg-holes.

The pants-type disposable diaper further comprises first stretchable elastic members extending in the longitudinal direction respectively and arranged between a longitudinal center line bisecting a width of the liquid-absorbent member and the opposite side edges of the liquid-absorbent member and secured in a stretched state between the liquid-absorbent member and the chassis so that a zone of the liquid-absorbent member extending between the first stretchable elastic members is deformed in a convexed shape toward a side facing a garment as the first stretchable elastic members contract.

In one embodiment of this invention, the first stretchable elastic members extend in a shape of substantially circular arcs which are convex toward the side edges of the liquid-absorbent member, respectively.

In another embodiment of this invention, the first stretchable elastic members extend across the crotch region into the front waist region.

In still another preferred embodiment of this invention, at least one of second stretchable elastic members extending in the waist-surrounding direction is arranged in the zone of the liquid-absorbent member and secured in a stretched state to at least the liquid-absorbent member out of the chassis and the liquid-absorbent member.

In another further embodiment of this invention, the liquid-absorbent member comprises a liquid-pervious topsheet facing a wearer's body, a liquid-impervious backsheet facing away from wearer' s body and a liquid-absorbent core interposed between the top- and backsheets with the first and second stretchable elastic members secured to the backsheet.
Fig. 1 is a perspective view of a typical embodiment of the disposable diaper according to this invention;
Fig. 2 is a partially cutaway perspective view showing a state of the diaper before shaped into pants-type one in Fig. 1;
Fig. 3 is a cross-sectional view taken along a line III - III in Fig. 1;
Fig. 4 is a cross-sectional view taken along a line IV - IV in Fig. 1;
Fig. 5 is a partially cutaway perspective view of another embodiment of the disposable diaper according to this invention;
Fig. 6 is a partially cutaway perspective view showing a state of the diaper before shaped into pants-type one in Fig. 5;
Fig. 7 is a cross-sectional view taken along a line VII - VII in Fig. 5; and
Fig. 8 is a cross-sectional view taken along a line VIII - VIII in Fig. 5.

Details of a pants-type disposable diaper will be more fully understood from the description given hereunder in reference to the accompanying drawings.

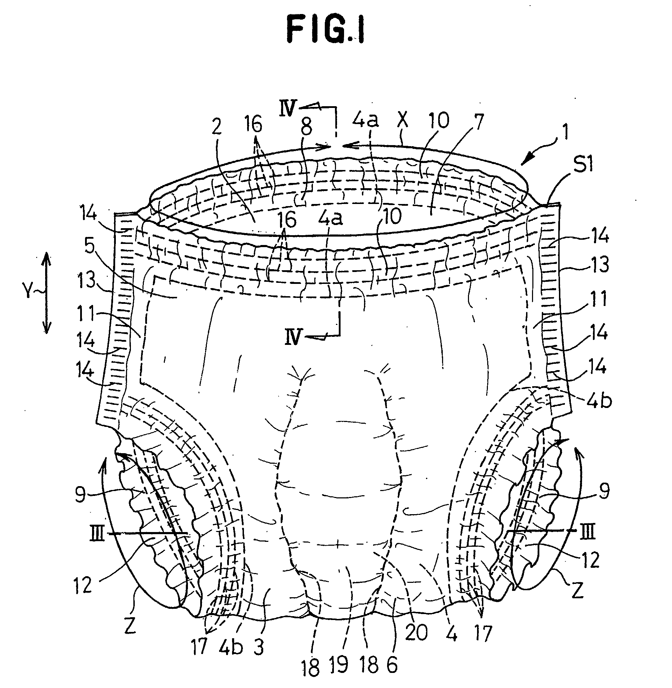

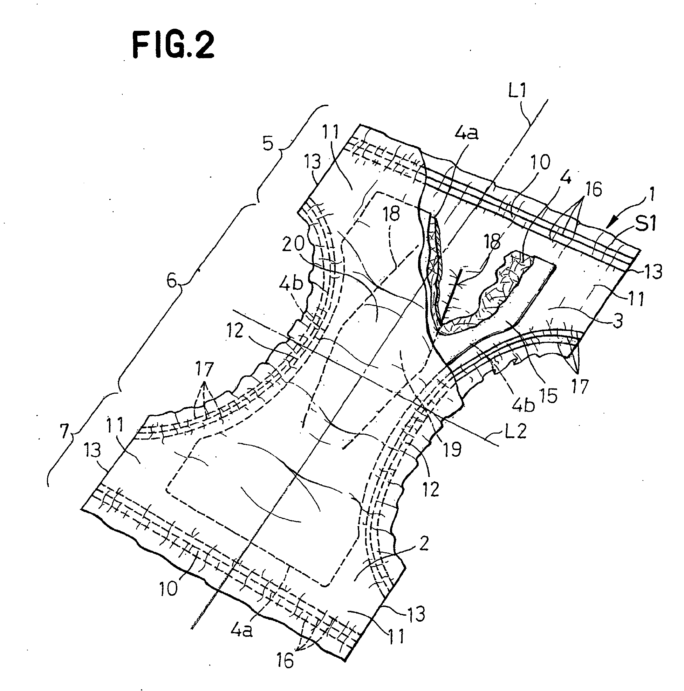

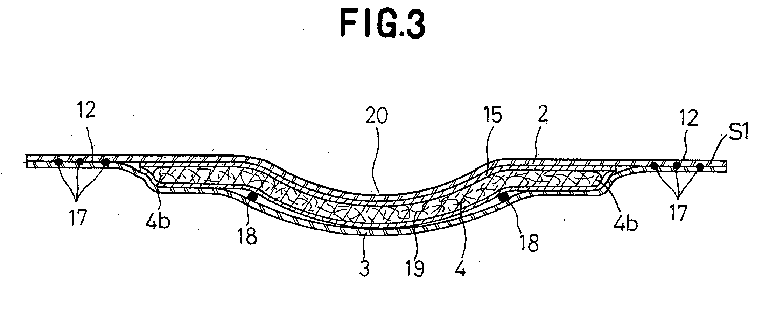

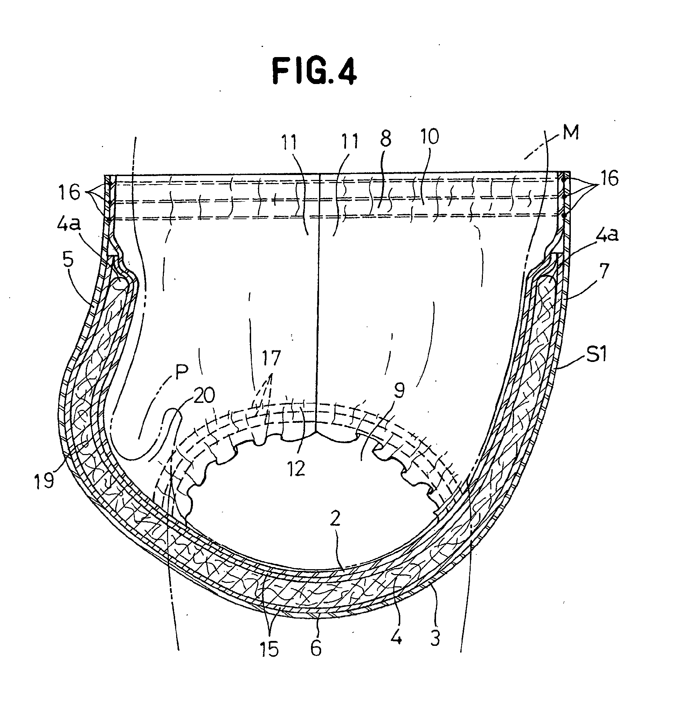

Fig. 1 is a perspective view of a typical embodiment of a disposable diaper 1 according to this invention, Fig. 2 is a perspective view showing the diaper in Fig. 1 as before shaped into pants-type one, Fig. 3 is a cross-sectional view taken along a line III - III in Fig. 1 and Fig. 4 is a cross-sectional view taken along a line IV - IV in Fig. 1. In Fig. 1, a waist-surrounding direction is indicated by an arrow X, a longitudinal direction is indicated by an arrow Y and a thigh-surrounding direction of a leg-hole is indicated by an arrow Z. In Fig. 4, a wearer M of the diaper 1 is indicated by imaginary lines. "Inner surfaces" of a liquid-pervious topsheet 2 and a liquid-impervious backsheet 3 should be understood to mean the surfaces thereof facing a core 4 and "outer surfaces" of these sheets 2, 3 should be understood to mean the surfaces thereof facing away from the core 4.

The diaper 1 basically comprises a liquid-pervious topsheet 2 facing a wearer's body, a liquid-impervious backsheet 3 facing a garment the wearer puts on and a liquid-absorbent core 4. The top- and backsheets 2, 3 form a chassis S1 and the core 4 may be referred to as a liquid-absorbent member. With respect to its configuration, the diaper 1 is composed of a front waist region 5 and a rear waist region 7 opposed to each other and a crotch region 6 extending between these waist regions 5, 7. The diaper 1 has a waist-hole 8 and a pair of leg-holes 9 arranged below the waist-hole 8.

As is shown in Fig. 2, the top- and backsheets 2, 3 respectively have waist-hole's peripheral portions 10 extending in the waist-surrounding direction and positioned in regions outside longitudinally opposite ends 4a of the core 4 extending in the front and rear waist regions 5, 7, waist's opposite side portions 11 extending in the longitudinal direction and positioned in regions outside transversely opposite side edges 4b of the core 4 extending in the front and rear waist regions 5, 7 and leg-holes' peripheral edge portions 12 extending in the thigh-surrounding direction and positioned in regions outside the side edges 4b of the core 4 extending in the crotch region 6. The top- and backsheets 2, 3 have respective inner surfaces joined together along the waist-hole's peripheral portions 10, the waist's opposite side portions 11 and the leg-holes' peripheral edge portions 12.

In the diaper 1 as is shown in Fig. 1, the top- and backsheets 2, 3 are overlaid together in the vicinity of side edges 13 contiguous to the respective side portions 11 of these sheets 2, 3 and joined together at a plurality of heat-sealing spots 14 arranged intermittently in the longitudinal direction along these side edges 13.

The core 4 extends across the crotch region 6 into the front and rear waist regions 5, 7. The core 4 is covered with a liquid-pervious sheet 15 and is joined to respective inner surfaces of the top- and backsheets 2, 3 with the liquid-pervious sheet 15 therebetween.

In the waist-hole's peripheral portions 10 are provided with a plurality of waist-surrounding elastic members 16 extending in the waist-surrounding direction and secured in a stretched state to these peripheral portions 10. In the leg-holes' peripheral portions 12 are provided with a plurality of thigh-surrounding elastic members 17 extending in the thigh-surrounding direction and secured in a stretched state to these peripheral portions 12. Both the waist-surrounding elastic members 16 and the thigh-surrounding elastic members 17 are interposed between the top- and backsheets 2, 3 and secured to respective inner surfaces of these sheets 2, 3. In the front waist region 5 and the crotch region 6 are provided with first stretchable elastic members 18 extending in the longitudinal direction and secured in a stretched state to the diaper 1.

As is seen in Fig. 2, the first stretchable elastic members 18 are respectively disposed in regions between a longitudinal center line L1 bisecting a width of the core 4 in the waist-surrounding direction and the opposite side edges 4b of the core 4. These first stretchable elastic members 18 take a shape of substantially circular arcs convexed toward adjacent side edges 4b extending across the crotch region 6 to the front waist region 5. These first stretchable elastic members 18 are interposed between the backsheet 3 and the core 4 and secured to the backsheet 3 and the liquid-pervious sheet 15 covering the core 4.

Contraction of the first stretchable elastic members 18 in the longitudinal direction deforms the core 4 to be convexed toward a side facing a garment a wearer puts on, that is, a side facing away from the wearer's body, in a zone 19 defined between the first stretchable elastic members 18. In the front waist region 5 and the crotch region 6 of the diaper 1, this zone 19 of the core 4 defines a penis receiving zone 20 adapted to receive penis P of the wearer M.

To shape the diaper 1 in Fig. 2 into the pants-type, the diaper 1 is folded back along a lateral center line L2 bisecting a length of the diaper 1 with the outer surface of the topsheet 2 opposed to itself and the top- and backsheets 2, 3 and the front and rear waist regions 5, 7 are connected to each other by bonding the sheets 2, 3 together in the vicinity of the side edges 13 contiguous to the respective waist side portions 11.

As is seen in Fig. 4, the penis P of the wearer M is received in the penis receiving zone 20 as the diaper is worn, so it is not likely that the penis P might be free to more within the diaper and particularly the penis P might turn toward any one of the leg-holes' peripheral edge portions 12. The diaper 1 ensures the penis P to be retained in the zone 20 defined in the transversely middle area of the front waist region 5 and the crotch region 6 in this manner ensuring urine to be discharged in this middle zone 20. Therefore, the diaper 1 ensures urine to be spread over and absorbed in the whole area of the core 4. There is no anxiety with the diaper 1 that urine might be discharged concentrated onto any one of the leg-holes' peripheral edge portions 12 and leaks sideways from these peripheral portions 12.

The first stretchable elastic members 18 may be secured to at least to the core 4 out of the backsheet 3 and the core 4. It is not essential for the first stretchable elastic members 18 to take a shape of substantially circular arcs which are convex toward the adjacent side edges 4b of the core 4 but it is also allowed for these first stretchable elastic members 18 to extend rather rectilinearly in the longitudinal direction. Alternatively, the first stretchable elastic members 18 may be extended from the front waist region 5 through the crotch region 6 to the rear waist region 7.

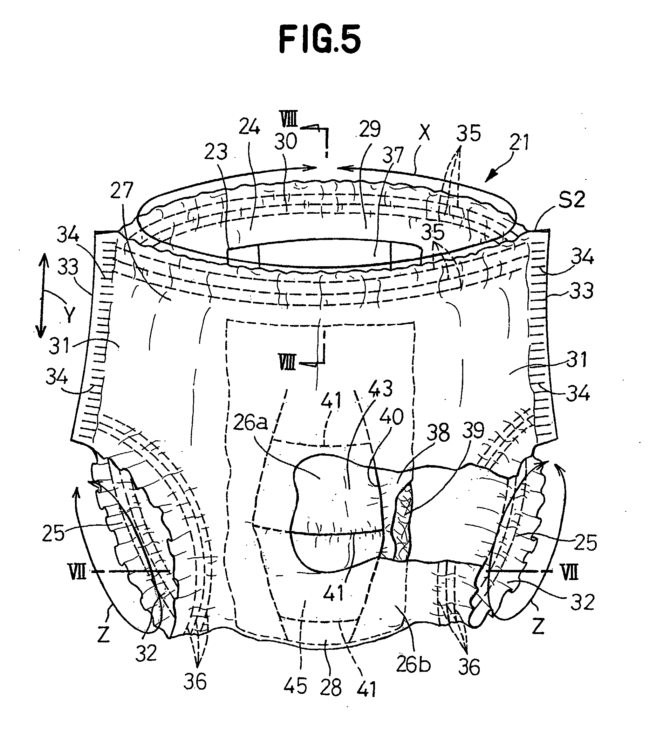

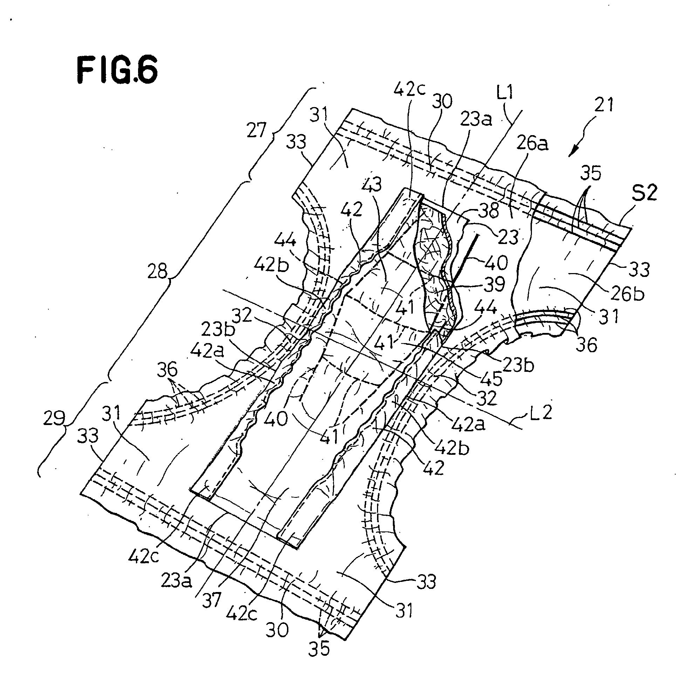

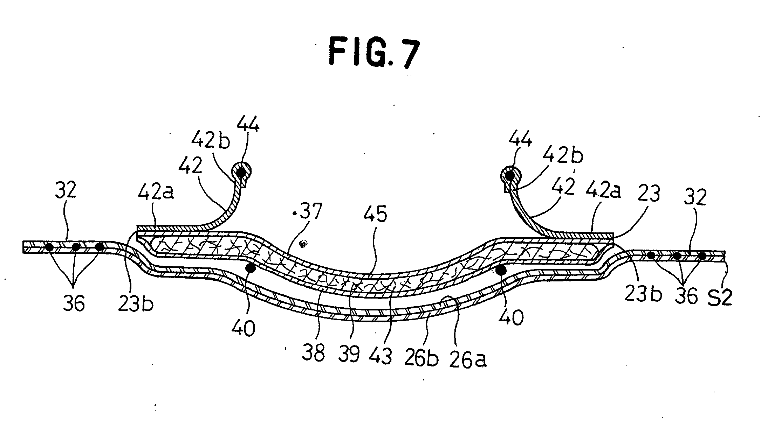

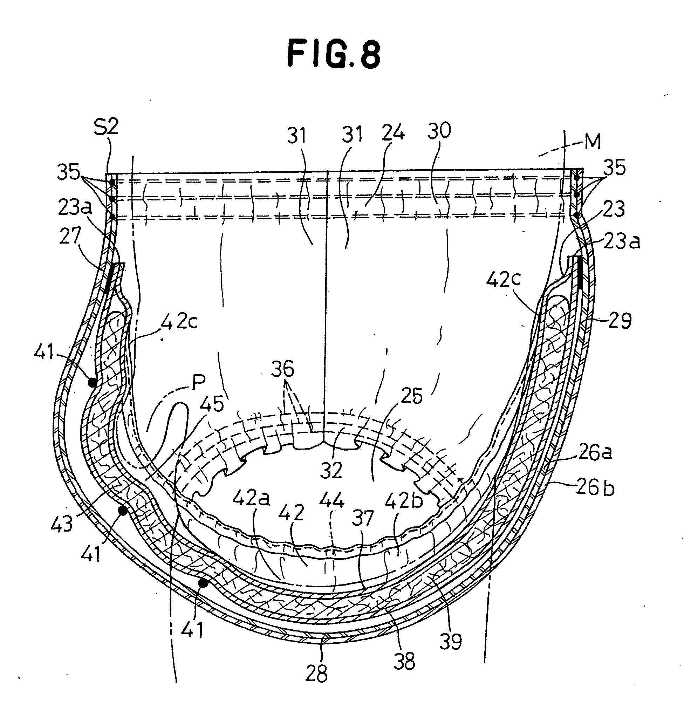

Fig. 5 is a perspective view of another embodiment of the disposable diaper 21 according to this invention, Fig. 6 is a perspective view showing the diaper 21 in Fig. 5 as before shaped into a pants-type one, Fig. 7 is a cross-sectional view taken along a line VII - VII in Fig. 5 and Fig. 8 is a cross-sectional view taken along a line VIII - VIII in Fig. 5. In Fig. 5, a waist-surrounding direction is indicated by an arrow X, a longitudinal direction is indicated by an arrow Y and a thigh-surrounding direction is indicated by an arrow Z. In Fig. 8, a wearer M is indicated by imaginary lines.

The diaper 21 basically comprises a chassis S2 having a waist-hole 24 and a pair of leg-holes 25 arranged below the waist-hole 24, and a liquid-absorbent member 23 attached to the inner side of the chassis S2.

The chassis S2 comprises substantially liquid-impervious sheets 26a, 26b overlaid each other. The chassis S2 is composed of front and rear waist regions 27, 29 opposed to each other and a crotch region 28 extending between these waist regions 27, 29. The sheets 26a, 26b of the chassis S2 have respective inner surfaces joined to each other.

The chassis S2 has waist-hole's peripheral portions 30 extending in the waist-surrounding direction and positioned in regions outside longitudinally opposite ends 23a of the liquid-absorbent member 23 extending in the front and rear waist regions 27, 29, waist's opposite side portions 31 extending in the longitudinal direction and positioned in regions outside transversely opposite side edges 23b of the liquid-absorbent member 23 extending in the front and rear waist regions 27, 29 and leg-holes' peripheral edge portions 32 extending in the thigh-surrounding direction and positioned in regions outside the side edges 23b of the liquid-absorbent member 23 extending in the crotch region 28. The chassis S2 is overlaid and joined together in the vicinity of side edges 33 respectively contiguous to the waist's opposite side portions 31 at a plurality of heat-sealing spots 34 arranged intermittently along the side edges 33 in the longitudinal direction.

In the waist-hole's peripheral portions 30 of the diaper 21 are provided with a plurality of waist-surrounding elastic members 35 extending in the waist-surrounding direction and secured in a stretched state to these peripheral portions 30. In the leg-holes' peripheral portions 32 of the diaper 21 are provided with a plurality of thigh-surrounding elastic members 36 extending in the thigh-surrounding direction and secured in a stretched state to these peripheral portions 32. Both the waist-surrounding elastic members 35 and the thigh-surrounding elastic members 36 are interposed between the sheets 26a, 26b of the chassis S2 and secured to respective inner surfaces of the sheets 26a, 26b.

The liquid-absorbent member 23 comprises a liquid-pervious topsheet 37 facing the wearer's body, a liquid-impervious backsheet 38 facing a garment the wearer puts on and a liquid-absorbent core 39 interposed between these top- and backsheets 37, 38. The core 39 is joined to respective inner surfaces of the top- and backsheets 37, 38. Similarly to that shown in Fig. 1, the core 39 is preferably covered with the liquid-pervious sheet.

The liquid-absorbent member 23 has a substantially rectangular shape in its developed state and extends across the crotch region 28 of the chassis S2 into the front and rear waist regions 27, 29. Peripheral portions of the top- and backsheets 37, 38 extending outwardly beyond a peripheral edge of the core 39 are overlaid and joined together. The liquid-absorbent member 23 is joined, in the vicinity of its longitudinally opposite ends 23a, to the sheet 26a extending in the front and rear waist regions 27, 29 by means of the backsheet 38.

First stretchable elastic members 40 extending in the longitudinal direction are secured in a stretched state to the liquid-absorbent member 23 and, a plurality of second stretchable elastic members 41 extending in parallel one to another in the waist-surrounding direction between these first stretchable elastic members 40 are secured in a stretched state to the liquid-absorbent member 23. In the vicinity of the side edges 23b of the liquid-absorbent member 23, a pair of substantially liquid-impervious lateral sheets 42 extending in the longitudinal direction are joined to the liquid-absorbent member 23.

As is seen in Fig. 6, the first stretchable elastic members 40 are respectively disposed in regions between a longitudinal center line L1 bisecting a width of the liquid-absorbent member 23 in the waist-surrounding direction and respective side edges 23b of the liquid-absorbent member 23 extending in the crotch region 28 and further into the front waist region 27 of the chassis S2. These first stretchable elastic members 40 take a shape of substantially circular arcs extending in convex toward adjacent side edges 23b. The second stretchable elastic members 41 are arranged in a zone 43 of the liquid-absorbent member 23 defined between the first stretchable elastic members 40 and spaced one from another by a given dimension in the longitudinal direction in the front waist region 27 and the crotch region 28 of the chassis S2. The first stretchable elastic members 40 and the second stretchable elastic members 41 are secured to the outer surface of the backsheet 38 but not to the sheet 26a forming the chassis S2. Alternatively, the second stretchable elastic members 41 may be extended to the side edges 23b of the liquid-absorbent member 23 and/or to the vicinity of the opposite end 23a positioned in the front waist region 27.

The lateral sheets 42 respectively have fixed side edge portions 42a joined to the liquid-absorbent member 23 in the vicinity of its side edges 23b and extending in the longitudinal direction, free side portions 42b extending in parallel to the fixed side portions 42a in the longitudinal direction and respective longitudinally opposite fixed end portions 42c collapsed inwardly in the transverse direction of the liquid-absorbent member 23 and joined in such a collapsed state to the liquid-absorbent member 23 in the vicinity of its respective ends 23a. The fixed side portions 42a and the fixed longitudinally opposite end portions 42c are joined to the outer surface of the topsheet 37. The free side portions 42b are respectively provided with stretchable elastic members 44 secured in a stretched state to these free side portions 42b. These elastic members 44 are wrapped with parts of the respective free side portions 42b.

Contraction of the first stretchable elastic members 40 in the longitudinal direction and contraction of the second stretchable elastic members 41 in the waist-surrounding direction deforms the liquid-absorbent member 23 in the zone 43 so that this zone 43 may be convexed toward a garment. In the front waist region 27 and the crotch region 28 of the diaper 21, this zone 43 of the liquid-absorbent member 23 defines a penis receiving zone 45 adapted to receive penis P of the wearer M.

To shape the diaper in Fig. 6 into the pants-type diaper 21, the chassis S2 and the liquid-absorbent member 23 are folded back along a lateral center line L2 with the outer surface of the topsheet 37 opposed to itself and the front and rear waist regions 27, 29 are connected to each other by joining the sheets 26a, 26b together in the vicinity of the side edges 33 contiguous to the respective waist side portions 31.

The penis P of the wearer M is received in the penis receiving zone 45 as the diaper 21 is worn, so it is not likely that the penis P might be protruded from the side edges 23b. In other words, urine is necessarily discharged onto the transversely middle zone in the front waist region 27 and the crotch region 28 of the diaper 21 and there is no anxiety that any amount of urine might leak sideways from one of the leg-holes' peripheral edge portions 32.

The liquid-absorbent member 23 is provided with, in addition to the first stretchable elastic members 40, the second stretchable elastic members 41, so contractile force of these elastic members 40, 41 can be further utilized to facilitate the liquid-absorbent member 23 to be deformed in the zone 43 toward the garment. The first and second stretchable elastic members 40, 41 are not secured to the sheet 26a forming the chassis S2 and therefore there is no possibility that a plurality of fine gathers might be formed on the chassis S2 as these elastic members 40, 41 contract.

The free side portions 42b of the respective lateral sheets 42 attached to the liquid-absorbent member 23 rise on the topsheet 37 as the stretchable elastic members 44 contract in the longitudinal direction. Consequently, the free side portions 42b of the respective lateral sheets 42 form barriers against bodily discharges and prevent bodily discharges from leaking sideways beyond the side edges 23b of the liquid-absorbent member 23.

A stock material for the topsheet 2, 37 may be selected from a group of materials including a fibrous nonwoven fabric treated to be hydrophilic, a thermoplastic film having a plurality of micro pores and a hydrophobic fibrous nonwoven fabric. The liquid-pervious sheet 15 may be formed by a tissue paper or a fibrous nonwoven fabric treated to be hydrophilic. The backsheets 3, 38 and the liquid-impervious sheets 26a, 26b may be formed using a material selected from a group of materials including a hydrophobic fibrous nonwoven fabric, a breathable but liquid-impervious plastic film, a composite nonwoven fabric consisting of two or more layers of hydrophobic fibrous nonwoven fabric laminated one with another and a composite sheet consisting of a hydrophobic nonwoven fabric and a breathable but liquid-impervious plastic film.

Nonwoven fabric may be selected from a group of materials including products obtained by spun lacing, needle punching, melt blowing, thermal bonding, spun bonding, chemical bonding and air-through processes. Component fibers of the nonwoven fabric may be selected from a group of materials including polyolefine-, polyester- and polyamide-based fibers and core-sheath-type and side-by-side-type conjugated fibers of polyethylene/polypropylene and polyethylene/polyester.

The cores 4, 39 are formed by a mixture of fluff pulp and super-absorbent polymer particles or a mixture of fluff pulp, super-absorbent polymer particles and thermoplastic synthetic resin fiber compressed to a desired thickness. The polymer particles may be selected from a group of materials consisting of a starch-based polymer, a cellulose-based polymer or a synthetic polymer.

Joining of sheets 2, 3, 26a, 26b, 37, 38, joining of the cores 4, 39 and securing of the elastic members 16, 17, 18, 35, 36, 40, 41, 44 may be carried out using a hot melt adhesive or welding technique such as heat-sealing or sonic-sealing.

The disposable diaper according to this invention has the advantageous effects that contraction of the first stretchable elastic members in the longitudinal direction deforms the liquid-absorbent member in the zone extending between the first stretchable elastic members so that this zone is deformed to be convexed toward the garment and defines the penis receiving zone. With this diaper, the wearer's penis is received in the penis receiving zone so it is not likely that the penis might be free to move within the diaper. Specifically, the wearer's penis is retained in the transversely middle zone of the front waist region and the crotch region. This diaper is effective to prevent the wearer's penis from turning toward any one of the leg-holes' peripheral edge portions. In this way, there is no anxiety that urine might be discharged concentrated onto any one of the leg-holes' peripheral edge portions and leak sideways from these peripheral edge portions.

In the embodiment of this invention according to which there are provided the second stretchable elastic members extending between the first stretchable elastic members in the waist-surrounding direction, contraction of the first stretchable elastic members in the longitudinal direction and contraction of the second stretchable elastic members in the waist-surrounding direction deform the predetermined zone of the liquid-absorbent member to be convexed toward the garment and this zone defines the penis receiving zone. In this diaper, the wearer's penis is received as the diaper is worn, so there is no anxiety that the wearer' s penis might turn toward any one of the leg-holes' peripheral edge portions and, in consequence, urine might leak beyond this peripheral edge portion. Contraction of the first stretchable elastic members cooperates with contraction of the second stretchable elastic members to facilitate the predetermined zone of the liquid-absorbent member to be convexed toward the garment.

## Claims

1. A pants-type disposable diaper comprising a chassis defining front and rear waist regions opposed to each other and a crotch region extending between said waist regions, and a liquid-absorbent member extending across said crotch region into said front and rear waist regions, said chassis having longitudinally opposite end portions lying outside longitudinally opposite ends of said liquid-absorbent member extending across said front and rear waist regions in a waist-surrounding direction and transversely opposite side portions lying outside transversely opposite side edges of said liquid-absorbent member extending across said front and rear waist regions in a longitudinal direction, and said front and rear waist regions being joined to each other in a vicinity of side edges contiguous to said opposite side portions forming a waist-hole and a pair of leg-holes, said pants-type disposable diaper further comprising:
first stretchable elastic members extending in said longitudinal direction respectively arranged between a longitudinal center line bisecting a width of said liquid-absorbent member and said opposite side edges of said liquid-absorbent member and secured in a stretched state between said chassis and said liquid-absorbent member so that a zone of said liquid-absorbent member extending between said first stretchable elastic members is deformed in a convexed shape toward a side facing away from a wearer's body as said first stretchable elastic members contract.

2. The diaper according to Claim 1, wherein said first stretchable elastic members extend in a shape of substantially circular arcs which are convex toward said side edges of said liquid-absorbent member, respectively.

3. The diaper according to Claim 1, wherein said first stretchable elastic members extend across said crotch region into said front waist region.

4. The diaper according to Claim 1, wherein at least one of second stretchable elastic members extending in said waist-surrounding direction is arranged in said zone of said liquid-absorbent member and secured in a stretched state between said chassis and said liquid-absorbent member.

5. The diaper according to Claim 1, wherein said liquid-absorbent member comprises a liquid-pervious topsheet facing a wearer' s body, a liquid-impervious backsheet facing away from the wearer's skin and a liquid-absorbent core interposed between said top- and backsheets and wherein said first and second stretchable elastic members are secured to said backsheet.
